(19) Europäisches Patentamt
European Patent Office
Office européen des brevets

(11) **EP 3 476 294 B1**

(12) **EUROPÄISCHE PATENTSCHRIFT**

(45) Veröffentlichungstag und Bekanntmachung des Hinweises auf die Patenterteilung:
**28.06.2023 Patentblatt 2023/26**

(21) Anmeldenummer: **17199026.0**

(22) Anmeldetag: **27.10.2017**

(51) Internationale Patentklassifikation (IPC):
**A61B 6/00** *(2006.01)*    **A61B 5/00** *(2006.01)*

(52) Gemeinsame Patentklassifikation (CPC):
**A61B 6/504;** A61B 5/0035; A61B 5/489; A61B 6/12

(54) **BESTIMMEN EINER PUNKTIONSPOSITION EINES GEFÄSSES**

DETERMINING OF A PUNCTURE POSITION OF A VESSEL

DÉTERMINATION D'UNE POSITION DE PONCTION D'UN VAISSEAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.05.2019 Patentblatt 2019/18**

(73) Patentinhaber: **Siemens Healthcare GmbH**
**91052 Erlangen (DE)**

(72) Erfinder:
• **Breininger, Katharina**
**91052 Erlangen (DE)**
• **Pfister, Marcus**
**91088 Bubenreuth (DE)**

(56) Entgegenhaltungen:
**DE-A1-102012 205 647    DE-A1-102015 202 287**
**US-A1- 2011 235 876**

• **GOEL V R ET AL: "Automated Vascular Geometric Analysis of Aortic Aneurysms", IEEE COMPUTER GRAPHICS AND APPLICATIONS, IEEE SERVICE CENTER, NEW YORK, NY, US, Bd. 28, Nr. 3, 1. Mai 2008 (2008-05-01), Seiten 76-86, XP011208296, ISSN: 0272-1716**

**Beschreibung**

Zur Unterstützung von Katheteranwendungen ist es bekannt, vorab aufgenommene erste Bilddatensätze zusammen mit zweiten Bilddatensätzen darzustellen, wobei die zweiten Bilddatensätze während der Katheteranwendung aufgenommen werden. Hierbei kann der erste Bilddatensatz insbesondere mit einer anderen Modalität als der zweite Bilddatensatz aufgenommen werden. Üblich ist beispielsweise die Kombination einer Computertomographie im Vorfeld der Katheteranwendung und einer Röntgenprojektion mittels eines C-Arm-Röntgengerätes während der Katheteranwendung.

Für die überlagerte Darstellung von ersten und zweiten Bilddatensätzen ist eine Registrierung notwendig und beispielsweise aus der Patentschrift US 8929631 B2 bekannt. Ist die Steifigkeit des Katheters jedoch sehr hoch, kann das Gefäß, in welches der Katheter eingeführt wird, verformt werden, so dass die Überlagerung des ersten und des zweiten Bilddatensatzes fehlerhaft ist (da der erste Bilddatensatz, im Gegensatz zum zweiten Bilddatensatz, nicht die Gefäßform während der Katheteranwendung darstellt).

Unter der Annahme, dass der Katheter sich innerhalb des Bildes befindet, ist aus der Druckschrift US 20150094567 A1 bekannt, den ersten Bilddatensatz basierend auf der Abbildung des Katheters in dem zweiten Bilddatensatz anzupassen. Aus der Druckschrift EP 17164593.0 ist es weiterhin bekannt, die Steifheit des Katheters in diese Anpassung einzubeziehen.

Da während einer Katheteruntersuchung mehrere zweite Bilddatensätze gewonnen werden, wird üblicherweise der Bildbereich der zweiten Bilddatensätze möglichst klein gewählt, um die Strahlendosis für den Patienten möglichst gering zu halten. Dies impliziert, dass der Katheter insbesondere nicht vollständig in den zweiten Bilddatensätzen abgebildet wird. Außerhalb des Bildbereiches der zweiten Bilddatensätze muss daher der Verlauf des Katheters extrapoliert werden, was mit Unsicherheiten verknüpft ist.

Ein Artikel von Goel et al. (GOEL V R ET AL: "Automated Vascular Geometrie Analysis of Aortic Aneurysms", IEEE COMPUTER GRAPHICS AND APPLICATIONS, Bd. 28, Nr. 3, Mai 2008, Seiten 76-86) befasst sich mit der automatisierten Analyse von Gefäßaneurysmen.

Es ist daher die Aufgabe der vorliegenden Erfindung, zusätzliche Informationen über den räumlichen Verlauf des Katheters bereitzustellen, welche eine genauere Extrapolation des Verlaufs des Katheters ermöglicht.

Die Aufgabe wird gelöst durch ein Verfahren zum Bestimmen einer Punktionsposition nach Anspruch 1, durch eine Positionsbestimmungseinheit nach Anspruch 9, durch eine bildgebende medizinische Vorrichtung nach Anspruch 11, durch ein Computerprogrammprodukt nach Anspruch 12, sowie durch ein computerlesbares Speichermedium nach Anspruch 13.

Hierbei bezeichnet die Punktionsposition einen Punkt in einem ersten Bilddatensatz, der die voraussichtliche Stelle des Eindringens des Katheters in ein Gefäß beschreibt. Da Katheter nur an wenigen ausgezeichneten Stellen eingeführt werden können, kann der Punktionspunkt alleine aufgrund der vorab aufgenommenen ersten Bilddaten bestimmt werden, und muss nicht während der Katheteranwendung bestimmt werden. Dieser Punktionspunkt kann dann beispielsweise neben der Extrapolation des Katheterverlaufs zusätzlich als Fixpunkt zur Anpassung einer Segmentierung des ersten Bilddatensatzes verwendet werden.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein Verfahren zum Bestimmen einer Punktionsposition eines Gefäßes, umfassend das Empfangen eines ersten Bilddatensatzes eines Untersuchungsbereichs mittels einer Schnittstelle, wobei der erste Bilddatensatz das Gefäß abbildet. Insbesondere verläuft das Gefäß im Untersuchungsbereich. Ein weiterer Schritt des erfindungsgemäßen Verfahrens ist das Bestimmen einer Gefäßlinie des Gefäßes basierend auf dem ersten Bilddatensatz mittels einer Recheneinheit. Ein weiterer Schritt des erfindungsgemäßen Verfahrens ist das Bestimmen eines Steigungsmaßes basierend auf der Gefäßlinie. Ein weiterer Schritt des erfindungsgemäßen Verfahrens ist das Bestimmen der Punktionsposition des Gefäßes basierend auf dem Steigungsmaß mittels der Recheneinheit.

Die Erfinder haben erkannt, dass Katheter üblicherweise an einer Stelle in Gefäße eingeführt werden, die am nächsten zur Oberfläche der Haut gelegen sind, da so möglichst wenig anderes Gewebes verletzt wird. Durch das Bestimmen basierend auf dem Steigungsmaß der Gefäßlinie kann die Punktionsposition daher sehr genau basierend auf dem ersten Bilddatensatz bestimmt werden.

Nach einem weiteren Aspekt der Erfindung umfasst das Verfahren den Verfahrensschritt des Bestimmens einer projizierten Gefäßlinie durch Projektion der Gefäßlinie entlang einer ersten Richtung mittels der Recheneinheit. Weiterhin ist das Steigungsmaß ein Steigungsmaß der projizierten Gefäßlinie bezüglich einer zweiten Richtung, wobei die zweite Richtung orthogonal zur ersten Richtung ist. Insbesondere ist die erste Richtung orthogonal zur Sagittalebene eines Patienten, und insbesondere ist die zweite Richtung orthogonal zur Transversalebene des Patienten. Mit anderen Worten spannen die erste Richtung und die zweite Richtung insbesondere die Frontalebene eines Patienten auf. Die Erfinder haben erkannt, dass bei einer projizierten Gefäßlinie, insbesondere bei einer zweidimensionalen projizierten Gefäßlinie, besonders einfach und effizient eine Steigungsmaß berechnet werden kann.

Nach einem weiteren Aspekt der Erfindung basiert das Steigungsmaß auf einem Tangentenvektor der Gefäßlinie. Insbesondere kann das Steigungsmaß auch auf mehreren Tangentenvektoren der Gefäßlinie in mehreren Punkten der Gefäßlinie basieren. Die Erfinder haben erkannt, dass basierend auf dem Tangentenvektor der Gefäßlinie deren Änderung und damit insbesondere ein Steigungsmaß besonders genau bestimmt werden können.

Nach einem weiteren möglichen Aspekt wird die Gefäßlinie des Gefäßes basierend auf einer Segmentierung des Gefäßes im ersten Bilddatensatz bestimmt. Die Erfinder haben erkannt, dass die Gefäßlinie besonders genau und effizient basierend auf einer bereits vorhandenen Segmentierung bestimmt werden kann. Insbesondere können hiermit auch manuelle Segmentierungen des Gefäßes im ersten Bilddatensatz als Ausgangspunkt für die Bestimmung der Gefäßlinie verwendet werden.

Nach einem weiteren möglichen Aspekt wird die Gefäßlinie als Grundlage für eine Segmentierung des Gefäßes im ersten Bilddatensatz bestimmt. In anderen Worten wird also zunächst eine Gefäßlinie bestimmt, und eine Segmentierung des Gefäßes im ersten Bilddatensatz könnte anschließend basierend auf der Gefäßlinie bestimmt werden. Die Erfinder haben erkannt, dass es durch ein solches Vorgehen nicht notwendig ist, die Segmentierung des Gefäßes im ersten Bilddatensatz zu bestimmen, ein solches Vorgehen also die Ausführung des Verfahrens schneller und kostengünstiger macht.

Nach einem weiteren Aspekt des Verfahrens basiert die Bestimmung der Punktionsposition auf einer Nullstelle des Steigungsmaßes. Die Erfinder haben erkannt, dass die Punktionsposition insbesondere nahe an einem Maximum oder einem Minimum der Gefäßlinie liegt, welche durch Bereiche ohne Steigung bzw. einem Steigungsmaß nahe 0 charakterisiert sind.

Nach einem weiteren Aspekt des Verfahrens umfasst dieses weiterhin das Bestimmen eines Krümmungsmaßes basierend auf der Gefäßlinie mittels der Recheneinheit, weiterhin basiert das Bestimmen der Punktionsposition des Gefäßes auf dem Krümmungsmaß. Die Erfinder haben erkannt, dass ein Krümmungsmaß besonders gut dazu geeignet ist, zwischen einem Maximum und einem Minimum der projizierten Gefäßlinie zu unterscheiden, um damit Fehler bei der Bestimmung der Punktionsposition zu vermeiden.

Nach einem weiteren möglichen Aspekt der Erfindung basiert das Bestimmen der Punktionsposition auf dem Vorzeichen des Krümmungsmaßes. Die Erfinder haben erkannt, dass es basierend auf dem Vorzeihen des Krümmungsmaßes besonders einfach und daher schnell möglich ist, zwischen einem Maximum und einem Minimum der projizierten Gefäßlinie zu unterscheiden.

Nach einem weiteren möglichen Aspekt der Erfindung ist das Krümmungsmaß ein Krümmungsmaß der projizierten Gefäßlinie bezüglich der zweiten Richtung. Die Erfinder haben erkannt, dass bei einer projizierten Gefäßlinie, insbesondere bei einer zweidimensionalen projizierten Gefäßlinie, besonders einfach und effizient eine Krümmungsmaß berechnet werden kann.

Nach einem weiteren möglichen Aspekt der Erfindung basiert das Krümmungsmaß auf einem Tangentenvektor der Gefäßlinie. Insbesondere kann das Krümmungsmaß auch auf mehreren Tangentenvektoren der Gefäßlinie in mehreren Punkten der Gefäßlinie basieren. Die Erfinder haben erkannt, dass basierend auf dem Tangentenvektor der Gefäßlinie deren Änderung und damit insbesondere ein Krümmungsmaß besonders genau bestimmt werden können.

Nach einem weiteren Aspekt der Erfindung ist das Steigungsmaß eine erste Ableitung und/oder das Krümmungsmaß eine zweite Ableitung. Insbesondere wenn das Steigungsmaß ein Steigungsmaß der projizierten Gefäßlinie ist, ist das Steigungsmaß eine erste Ableitung der projizierten Gefäßlinie nach der zweiten Richtung. Insbesondere wenn das Krümmungsmaß ein Krümmungsmaß der projizierten Gefäßlinie ist, ist das Krümmungsmaß eine zweite Ableitung der projizierten Gefäßlinie nach der zweiten Richtung. Insbesondere wenn das Steigungsmaß auf einem Tangentenvektor der Gefäßlinie basiert, basiert das Steigungsmaß auf einer ersten Ableitung nach dem Kurvenparameter. Insbesondere wenn das Krümmungsmaß auf einem Tangentenvektor der Gefäßlinie basiert, basiert das Krümmungsmaß auf einer zweiten Ableitung nach dem Kurvenparameter. Eine erste und/oder eine zweite Ableitung kann auch auf nur einem Teil der Gefäßlinie bzw. der projizierten Gefäßlinie berechnet werden. Die Erfinder haben erkannt, dass sich die erste Ableitung besonders gut als Steigungsmaß eignet, und dass sich die zweite Ableitung besonders gut als Krümmungsmaß eignet, und somit eine besonders genaue Bestimmung der Punktionsposition möglich ist.

Nach einem weiteren Aspekt der Erfindung basiert das Bestimmen der Punktionsposition weiterhin auf einem Extremum der Gefäßlinie, wobei sich die Koordinaten des Extremums von den Koordinaten der Punktionsposition unterscheiden. In anderen Worten sind das Extremum und die Punktionsposition zwei unterschiedliche Punkte der Gefäßlinie. Insbesondere ist das Extremum ein Extremum der projizierten Gefäßlinie. Die Erfinder haben erkannt, dass basierend auf einem Extremum eine genauere und weniger fehleranfällige Bestimmung der Punktionsposition möglich ist, da dieses Extremum weitere Informationen über den geometrischen Verlauf der Gefäßlinie umfasst.

Nach einem weiteren Aspekt der Erfindung ist die Punktionsposition bezüglich des Extremums distal oder proximal gelegen. Insbesondere kann die Punktionsposition bezüglich des Extremums distal gelegen sein. Insbesondere kann die Punktionsposition bezüglich des Extremums proximal gelegen sein. Hierbei ist die Punktionsposition distal gelegen, wenn sie im Verlauf des Gefäßes weiter von der Körpermitte bzw. dem Herzen entfernt ist als das Extremum. Weiterhin ist hierbei die Punktionsposition proximal gelegen, wenn sie im Verlauf des Gefäßes näher an der Körpermitte bzw. dem Herzen gelegen ist als das Extremum. Die Erfinder haben erkannt, dass durch anatomische Restriktionen nur ein kleinerer Teil der projizierten Gefäßlinie analysiert werden muss, dies führt einerseits zu einer schnelleren Ausführung des Verfahrens, andererseits wird hierdurch das Verfahren weniger fehleranfällig.

Nach einem weiteren möglichen Aspekt der Erfindung ist das Gefäß eine Arterie oder eine Vene. Die Erfinder haben erkannt, dass das Verfahren für Arterien und Venen aufgrund ihrer Geometrie und ihrer Materialeigenschaften besonders effizient angewendet werden kann.

Nach einem weiteren Aspekt der Erfindung wird beim Bestimmen der Gefäßlinie weiterhin eine erste Segmentierung des Gefäßes bestimmt, weiterhin umfasst das Verfahren den Schritt des Empfangens eines zweiten Bilddatensatzes des Untersuchungsbereichs mittels der Schnittstelle; weiterhin umfasst das Verfahren den Schritt des Bestimmens einer zweiten Segmentierung des Gefäßes basierend auf der ersten Segmentierung, dem Punktionspunkt des Gefäßes und dem zweiten Bilddatensatz mittels der Recheneinheit. Die Erfinder haben erkannt, dass unter Verwendung des Punktionspunktes die zweite Segmentierung genauer und weniger fehleranfällig bestimmt werden kann, da durch den Punktionspunkt ein zusätzlicher Fixpunkt für die Anpassung der Segmentierung vorliegt.

Nach einem weiteren Aspekt der Erfindung bildet der zweite Bilddatensatz ein medizinische Instrument im Untersuchungsbereich ab; weiterhin umfasst das Verfahren den Schritt des Bestimmens einer ersten Instrumentenposition basierend auf dem zweiten Bilddatensatz; weiterhin umfasst das Verfahren den Schritt des Extrapolierens einer zweiten Instrumentenposition basierend auf der ersten Instrumentenposition und der Punktionsposition; wobei der Schritt des Bestimmens der zweiten Segmentierung derart erfolgt, dass die erste Instrumentenposition und die zweite Instrumentenposition innerhalb der zweiten Segmentierung des Gefäßes angeordnet sind. Bei einem medizinischen Instrument handelt es sich insbesondere um ein längliches Instrument zur Untersuchung eines Gefäßes. Bei dem medizinischen Instrument kann es sich insbesondere um einen Katheter handeln, in diesem Fall handelt es sich bei der ersten Instrumentenposition um eine erste Katheterposition und bei der zweiten Instrumentenposition um eine zweite Katheterposition. Die Erfinder haben erkannt, dass basierend auf einer extrapolierten Instrumentenposition die zweite Segmentierung besonders effizient bestimmt werden kann.

Die Erfindung betrifft auch eine Positionsbestimmungseinheit zum Bestimmen einer Punktionsposition, umfassend folgende Einheiten:

- Schnittstelle, ausgebildet zum Empfangen eines ersten Bilddatensatzes eines Untersuchungsbereichs, wobei der erste Bilddatensatz das Gefäß abbildet,
- Recheneinheit, ausgebildet zum Bestimmen einer Gefäßlinie des Gefäßes basierend auf dem ersten Bilddatensatz,

 weiterhin ausgebildet zum Bestimmen eines Steigungsmaßes basierend auf der Gefäßlinie,
 weiterhin ausgebildet zum Bestimmen der Punktionsposition des Gefäßes basierend auf dem Steigungsmaß.

Eine solche Positionsbestimmungseinheit kann insbesondere dazu ausgebildet sein die zuvor beschriebenen erfindungsgemäßen Verfahren und ihre Aspekte auszuführen. Die Positionsbestimmungseinheit ist dazu ausgebildet diese Verfahren und ihre Aspekte auszuführen, indem die Schnittstelle und die Recheneinheit ausgebildet sind die entsprechenden Verfahrensschritte auszuführen.

Die Erfindung betrifft weiterhin eine bildgebende medizinische Vorrichtung umfassend eine erfindungsgemäße Positionsbestimmungseinheit. Die bildgebende medizinische Vorrichtung ist insbesondere dazu ausgebildet, zweite Bilddatensätze aufzunehmen. Die Bildgebung der bildgebenden medizinischen Vorrichtung kann insbesondere auf ionisierenden Strahlen, insbesondere auf Röntgenstrahlen basieren. Die bildgebende medizinische Vorrichtung kann insbesondere ein C-Bogen-Röntgengerät sein.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Positionsbestimmungseinheit auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Ein Bilddatensatz umfasst zumindest ein Bild, weiterhin kann ein Bilddatensatz noch Metadaten enthalten. Ist das Bild das Ergebnis einer bildgebenden medizinischen Untersuchung, dann können die Metadaten insbesondere den Namen, das Alter und/ oder das Geschlecht des Patienten umfassen, welcher der bildgebenden medizinischen Untersuchung unterzogen wurde. Ein n-dimensionaler Bilddatensatz umfasst zumindest ein n-dimensionales Bild. Insbesondere kann ein Bilddatensatz auch identisch mit einem Bild sein, insbesondere kann ein n-dimensionaler Bilddatensatz identisch mit einem n-dimensionalen Bild sein. Ein Bild kann insbesondere das Ergebnis einer bildgebenden medizinischen Untersuchung mittels ionisierender Strahlen, insbesondere mittels Röntgenstrahlen sein. In diesem Fall umfasst ein (n-dimensionales) Bild eine Menge von (n-dimensionalen) Voxeln, wobei jedem der Voxel ein Intensitätswert zugeordnet ist, der auf einem Röntgenabsorptionskoeffizienten basiert. Ein zweidimensionaler Voxel wird auch als Pixel bezeichnet.

Der erste Bilddatensatz ist insbesondere ein zweidimensionaler oder ein dreidimensionaler Bilddatensatz, der zweite Bilddatensatz ist insbesondere ein zweidimensionaler Bilddatensatz. Der erste Bilddatensatz kann insbesondere mit einer ersten bildgebenden medizinischen Vorrichtung bestimmt worden sein, der zweite Bilddatensatz kann insbesondere mit einer zweiten bildgebenden medizinischen Vorrichtung bestimmt worden sein.

Als Segmentierung bezeichnet man die Erzeugung von zusammenhängenden Strukturen in einem Bilddatensatz durch Zusammenfassung benachbarter Pixel oder Voxel, die eine gemeinsame Eigenschaft aufweisen. Insbesondere werden Pixel oder Voxel, die Teile des Bildes einer gleichen Körperregion sind, einer gemeinsamen zusammenhängenden Struktur im Bilddatensatz zugeordnet. Zur Segmentierung können bekannte Methoden der Bildverarbeitung wie kantenorientierte Verfahren, regionenorientierte Verfahren, modellbasierte Verfahren oder texturorientierte Verfahren zum Einsatz kommen. Weiterhin kann der Segmentierung von Gefäßstrukturen voraus gehen, dass eine oder mehrere Zentrallinien für Gefäße bestimmt werden. Die Segmentierung kann dann insbesondere auf den bestimmten Zentrallinien beruhen.

Insbesondere unterteilt eine Segmentierung eines Gefäßes den ersten Bilddatensatz in einen ersten Bereich, der dem Gefäß entspricht, und einem zweiten Bereich, der nicht dem Gefäß entspricht. Insbesondere kann dabei jedem Voxel des ersten Bilddatensatzes ein Wert zugewiesen werden, wobei der Wert darauf basiert, ob der zugehörige Voxel das Gefäß abbildet oder nicht. Beispielsweise kann den Voxeln, die das Gefäß abbilden, der Wert 1 zugewiesen werden, und den restlichen Voxeln der Wert 0. Ist der erste Bilddatensatz ein zweidimensionaler Bilddatensatz, so sind die erste und die zweite Segmentierung insbesondere jeweils zweidimensionale Segmentierungen. Ist der erste Bilddatensatz ein dreidimensionaler Bilddatensatz, so sind die erste und die zweite Segmentierung insbesondere jeweils dreidimensionale Segmentierungen. Eine Segmentierung kann also insbesondere auch als Bilddatensatz aufgefasst werden, der die gleiche Ausdehnung aufweist wie der erste Bilddatensatz. Die zweite Segmentierung ist hierbei insbesondere keine Segmentierung des zweiten Bilddatensatzes, sondern entspricht der realen Gefäßposition während der Untersuchung mit dem medizinischen Instrument (die von der Gefäßposition im ersten Bilddatensatz abweichen kann). Anstatt "erste Segmentierung" kann daher auch "vorab berechnete Segmentierung" verwendet werden, anstatt "zweite Segmentierung" kann daher auch "modifizierte Segmentierung" verwendet werden. Anstatt des Wortes "Segmentierung" kann auch das Wort "Gefäßstruktur" verwendet werden.

Eine Gefäßlinie ist insbesondere eine Menge von Punkten im ersten Bilddatensatz, wobei jeder Punkt der Menge von Punkten innerhalb des Gefäßes liegt. In anderen Worten wird jeder Punkt der Menge von Punkten durch eine Segmentierung des Gefäßes dem Gefäß zugeordnet. Die Menge von Punkten kann hierbei diskret und kontinuierlich sein. Ist die Gefäßlinie eine diskrete Menge von Punkten, kann die Gefäßlinie insbesondere durch die Angabe der Koordinaten aller Punkte gegeben sein. In diesem Fall kann ein Kurvenparameter durch eine Reihenfolge der Punkte gegeben sein, beispielsweise als Index. Ein Punkt der diskreten Menge von Punkten kann mit einem Pixel bzw. Voxel übereinstimmen, er kann aber auch unabhängig von einem Pixel bzw. Voxel des ersten Bilddatensatzes sein. Ist die Gefäßlinie eine kontinuierliche Menge von Punkten, kann eine Gefäßlinie auch durch eine von einem (insbesondere kontinuierlichen) Kurvenparameter abhängige (optional stetig und/oder differenzierbare) Kurve im ersten Bilddatensatz definiert sein. Insbesondere kann die Gefäßlinie auch die Zentrallinie des Gefäßes sein.

Eine projizierte Gefäßlinie ist eine Projektion der Gefäßlinie bezüglich einer ersten Richtung. Ist der erste Bilddatensatz ein zweidimensionaler Bilddatensatz, dann ist die projizierte Gefäßlinie insbesondere identisch mit der Gefäßlinie. In anderen Worten ist in diesem Fall der erste Bilddatensatz in zwei Richtungen ausgedehnt, die jeweils orthogonal zur ersten Richtung sind, und die Projektion entspricht einer Identitätsabbildung.

Die Punktionsposition ist insbesondere durch die Angaben von Koordinaten im ersten Bilddatensatz gegeben. Demnach kann eine Punktionsposition auch durch die Angabe eines Punktes einer Gefäßlinie angegeben werden, denn ein Punkt einer Gefäßlinie können Koordinaten im dreidimensionalen Bilddatensatz zugeordnet werden. Alternativ kann die Punktionsposition auch durch die Angabe eines Voxels im ersten Bilddatensatz charakterisiert sein.

Eine Instrumentenposition bezeichnet eine Position eines Teils bzw. eines Punktes des medizinischen Instrumentes insbesondere im ersten Bilddatensatz. Ist der erste Bilddatensatz ein zweidimensionaler Bilddatensatz, dann ist die Instrumentenposition insbesondere eine zweidimensionale Position. Ist der erste Bilddatensatz ein dreidimensionaler Bilddatensatz, dann ist die Instrumentenposition insbesondere eine dreidimensionale Instrumentenposition. Eine erste Instrumentenposition kann insbesondere basierend auf dem zweiten Bilddatensatz bestimmt werden. Insbesondere wenn das medizinische Instrument ein Katheter ist, kann "Katheterposition" synonym mit "Instrumentenposition" verwendet werden.

Im Folgenden wird die Erfindung anhand der in den Figuren dargestellten Ausführungsbeispiele näher beschrieben und erläutert.

Fig. 1    zeigt eine schematisch Darstellung des Beckenbereichs eines Patienten,

Fig. 2    zeigt einen vergrößerten Ausschnitt der schematischen Darstellung des Beckenbereichs des Patienten,

Fig. 3    zeigt das Prinzip einer Überlagerung eines ersten Bilddatensatzes und eines zweiten Bilddatensatzes während einer Katheteranwendung,

Fig. 4    zeigt eine weitere Überlagerung eines ersten Bilddatensatzes und eines zweiten Bilddatensatzes während einer Katheteranwendung,

Fig. 5    zeigt eine weitere Überlagerung eines ersten Bilddatensatzes und eines zweiten Bilddatensatzes während der Katheteranwendung,

Fig. 6    zeigt ein Flussdiagramm eines ersten Ausführungsbeispiels des erfindungsgemäßen Verfahrens zum Bestimmen einer Punktionsposition,

Fig. 7    zeigt eine projizierte Gefäßlinie, ein Steigungsmaß und ein Krümmungsmaß,

Fig. 8    zeigt ein Flussdiagramm eines zweiten Ausführungsbeispiels des erfindungsgemäßen Verfahrens zum Bestimmen einer Punktionsposition,

Fig. 9    zeigt eine Positionsbestimmungseinheit 90 zur Bestimmung einer Punktionsposition,

Fig. 10   zeigt experimentelle Daten, die sich aus der Anwendung des dargestellten Verfahrens zur Bestimmung einer Punktionsposition ergeben.

Die Figuren zeigen die Anwendung der Erfindung auf das Gefäß der äußeren Beckenarterie (der lateinische Fachbegriff ist "Arteria iliaca externa") bzw. deren Fortsetzung, die Oberschenkelarterie (der lateinische Fachbegriff ist "Arteria femoralis"). Ein Zugang über diese Arterien kann insbesondere zur Behandlung eines abdominellen Aorten-Aneurysmas (kurz AAA) mittels eines Stentgrafts (Gefäßprothese) genutzt werden. Die Erfindung kann aber auch auf andere Gefäße angewendet werden, beispielsweise die Schlüsselbeinvene (der lateinische Fachbegriff ist "vena subclavia"), die Unterschlüsselbeinarterie (der lateinische Fachbegriff ist "arteria subclavia"), die Ellenbeugenvene (der lateinische Fachbegriff ist "vena cubitalis"), die Oberarmarterie (der lateinische Fachbegriff ist "arteria brachialis"), die Speichenarterie (der lateinische Fachbegriff ist "arteria radialis") und die Halsader bzw. Drosselvene (der lateinische Fachbegriff ist "vena jugularis"). Weiterhin kann die Erfindung auch zur Berechnung von Punktionspunkten für die Periduralanästhesie oder die Spinalanästhesie angewendet werden.

Die Erfindung kann insbesondere dadurch auf andere Gefäße angewendet werden, weil Zugangspunkte zu Gefäßen vorteilhafterweise nahe an der Oberfläche eines Patienten gewählt werden, um einerseits das Gefäß beim Einführen eines medizinischen Instrumentes auch tatsächlich zu treffen, und andererseits die Verletzungen des umliegenden Gewebes so klein wie möglich zu halten.

Die im Zusammenhang mit der äußeren Beckenarterie und der Oberschenkelarterie beschriebenen vorteilhaften Aus- und Weiterbildungen sowie alternative Merkmale können auch auf die Anwendung der Erfindung auf andere Gefäße übertragen werden.

Weiterhin beschreiben die Figuren die Erfindung in Bezug auf die Anwendung eines Katheters 36. Die beschriebenen vorteilhaften Aus- und Weiterbildungen sowie alternative Merkmale können auch auf die Anwendung eines anderen medizinischen Instrumentes übertragen werden, insbesondere auf ein länglich ausgebildetes medizinisches Instrument.

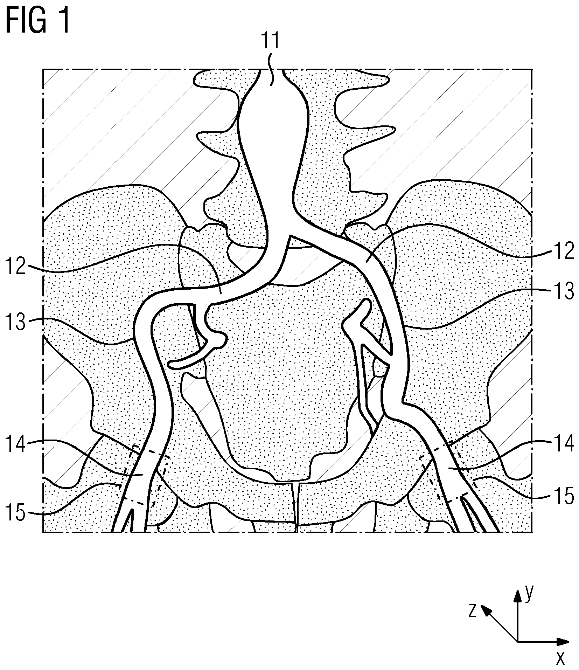

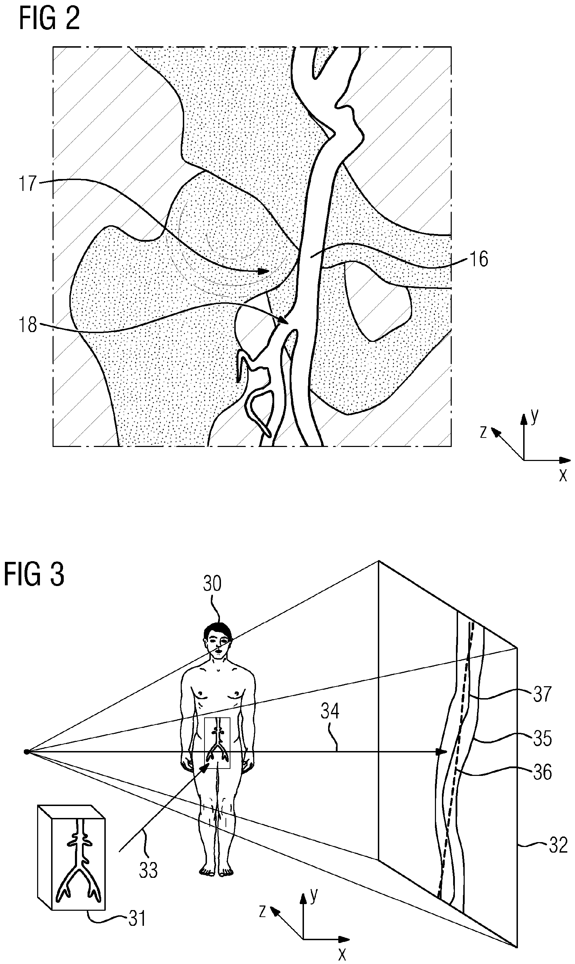

Fig. 1 zeigt eine schematisch Darstellung des Beckenbereichs eines Patienten 30, insbesondere die Hüftknochen und arterielle Gefäße, Fig. 2 zeigt einen vergrößerten Ausschnitt dieser Darstellung. In der Fig. 1 sind die Bauchaorta 11 (der lateinische Fachbegriff ist "Aorta abdominalis"), die beiden gemeinsame Darmbeinarterien 12 (der lateinische Fachbegriff ist "Arteria iliaca communis", bzw. in der Mehrzahl "Arteriae iliacae communes"), die beiden äußeren Beckenarterien 13 (der lateinische Fachbegriff ist "Arteria iliaca externa", bzw. in der Mehrzahl "Arteriae iliacae externae") sowie die beiden Oberschenkelarterien 14 (der lateinische Fachbegriff ist "Arteria femoralis", bzw. in der Mehrzahl "Arteriae femorales") abgebildet. Weiterhin sind die zwei Zugangsbereiche 15 dargestellt, in denen der Zugangspunkt 16 eines Katheters 36 zur Oberschenkelarterie 14 lokalisiert ist.

Als Zugangspunkt 16 zur Oberschenkelarterie 14 wird der oberflächennächste Punkt der Oberschenkelarterie 14 verwendet, der sich im Regelfall ca. 2 bis 5 cm unter der Haut des Patienten 30 befindet. Dieser Zugangspunkt 16 ist durch einen tastbaren Puls gut lokalisierbar, und liegt ca. 1 bis 2 cm distal des Leistenbandes (der lateinische Fachbegriff ist "Ligamentum inguinale") neben dem Kopf 17 des Oberschenkelknochens (der lateinische Fachbegriff ist "Caput ossis femoris"). Weiterhin ist in Fig. 2 die Bifurkation 18 der Oberschenkelarterie (bzw. die Abzweigung der tiefen Oberschenkelarterie, lateinisch "Arteria profunda femoris") dargestellt, die distal vom Zugangspunkt 16 liegt.

Fig. 3 zeigt das Prinzip einer Überlagerung eines ersten Bilddatensatzes 31 und eines zweiten Bilddatensatzes 32 während einer Katheteranwendung, wobei der erste Bilddatensatz 31 vor der Katheteranwendung bestimmt wurde, und wobei der zweite Bilddatensatz 32 während der Katheteranwendung bestimmt wurde. Im dargestellten Ausführungsbeispiel ist der erste Bilddatensatz 31 ein dreidimensionaler Bilddatensatz, der mittels einer Computertomographie bestimmt wurde. Alternativ kann der erste Bilddatensatz 31 auch ein dreidimensionaler Bilddatensatz sein, der beispielsweise mittels einer 3D-Rotationsangiographie bestimmt wurde. Im dargestellten Ausführungsbeispiel ist der zweite Bilddatensatz 32 weiterhin ein zweidimensionaler Röntgen-Bilddatensatz.

Der erste Bilddatensatz 31 ist hierbei mit dem Patienten 30 registriert 33, und kann durch eine Projektion 34 gemeinsam mit dem zweiten Bilddatensatz 32 dargestellt werden. In anderen Worten sind der erste Bilddatensatz 31 und der zweite Bilddatensatz 32 miteinander registriert. Im dargestellten Ausführungsbeispiel wird hierbei die Projektion einer ersten Segmentierung 35 des ersten Bilddatensatzes 31 dem zweiten Bilddatensatz 32 überlagert. Der zweite Bilddatensatz 32 umfasst hierbei auch das Bild des Katheters 36. Die Projektion des ersten Bilddatensatzes 31 und/oder die Projektion der ersten Segmentierung 35 des ersten Bilddatensatzes 31 können insbesondere auch als "virtuelles Bild" oder als "virtuelles Röntgenbild" bezeichnet werden, der zweite Bilddatensatz kann insbesondere auch als "reales Bild" oder als "reales Röntgenbild" bezeichnet werden.

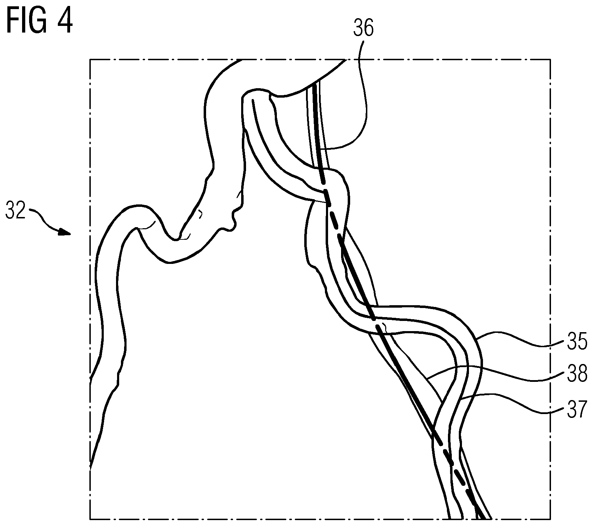

Fig. 4 zeigt eine weitere Überlagerung eines ersten Bilddatensatzes 31 und eines zweiten Bilddatensatzes 32 während einer Katheteranwendung. Die dargestellte Situation zeigt einen Katheter 36 mit hoher Steifigkeit, so dass das Gefäß (hier die gemeinsame Darmbeinarterien 12 und die externe Hüftarterie 13) durch den eingeführten Katheter 36 verformt wird. Dadurch verläuft der Katheter 36 im zweiten Bilddatensatz 32 nicht mehr durch die projizierte erste Segmentierung 35 (da die erste Segmentierung 35 basierend auf dem ersten Bilddatensatz 31 bestimmt wurde, der vor der Katheteranwendung aufgezeichnet wurde). In dieser Situation ist es aufgrund dieser Abweichungen nicht mehr möglich, den ersten Bilddatensatz 31 bzw. die erste Segmentierung 35 zur Unterstützung der Katheteranwendung zu verwenden. Weiterhin ist im zweiten Bilddatensatz 32 das Bild 38 des Gefäßes, in welcher der Katheter 36 verläuft, dargestellt.

Da davon ausgegangen werden kann, dass das Gefäß (hier die gemeinsame Darmbeinarterien 12 und die externe Hüftarterie 13) sich an die Form des Katheters 36 anpasst, kann basierend auf der Position des Katheters 36 im zweiten Bilddatensatz 32 durch Verformung der ersten Segmentierung 35 eine zweite Segmentierung bestimmt werden, die den Verlauf des Gefäßes im ersten Bilddatensatz 31 an die realen Gegebenheiten anpasst.

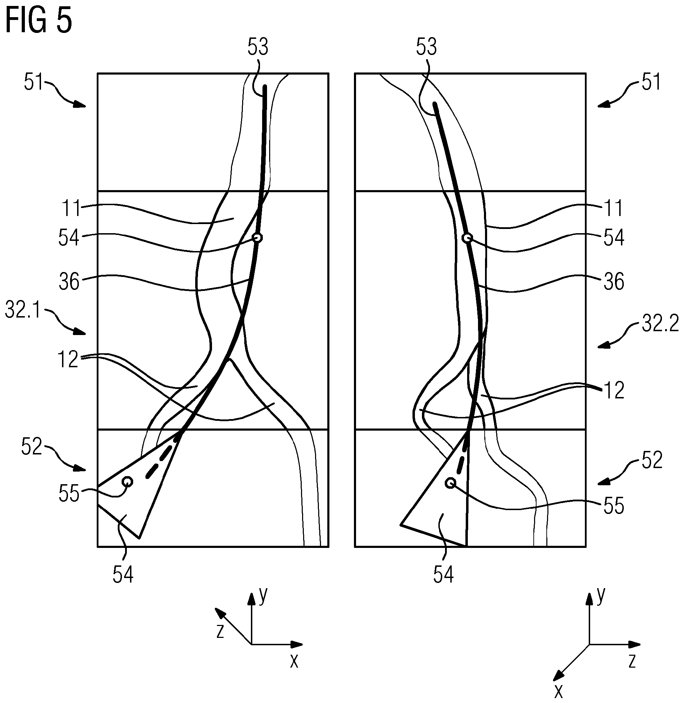

Fig. 5 zeigt eine weitere Überlagerung eines ersten Bilddatensatzes 31 und eines zweiten Bilddatensatzes 32 während einer Katheteranwendung, welcher eine Aortenbifurkation abbildet. Im dargestellten Ausführungsbeispiel umfasst der zweite Bilddatensatz 32 ein erstes zweidimensionales Bild 32.1 und ein zweites zweidimensionales Bild des Untersuchungsbereichs, wobei das erste zweidimensionale Bild 32.1 und das zweite zweidimensionale Bild 32.2 aus unterschiedlichen Projektionswinkeln aufgenommen wurden. Um die Röntgendosis für den Patienten 30 möglichst gering zu halten, wird das Sichtfeld des zweiten Bilddatensatzes 32 möglichst klein gehalten. Insbesondere wird der Katheter 36 also nicht vollständig im zweiten Bilddatensatz 32 dargestellt.

Im dargestellten Ausführungsbeispiel ergeben sich ein proximaler Bereich 51 und ein distaler Bereich 52, in denen der Katheter 36 nicht dargestellt werden kann, wobei der proximale Bereich 51 und der distale Bereich 52 aber im ersten Bilddatensatz 31 dargestellt sind. Da die Steifheit der Gefäße üblicherweise mit zunehmender Proximalität (d.h. Nähe zur Körpermitte bzw. zum Herzen) zunimmt, kann davon ausgegangen werden, dass der Katheter 36 im proximalen Bereich 51 das Gefäß nicht hinreichend verformen kann, daher kann der proximale Verlauf 53 des Katheters 36 durch den aus dem ersten Bilddatensatz 31 bekannten Verlauf des Gefäßes bestimmt bzw. approximiert werden. Im distalen Bereich 52 ist dies aber aufgrund der geringeren Steifigkeit des Gefäßes nicht möglich, es ergibt sich im distalen Bereich aufgrund der Flexibilität des Katheters 36 ein Unsicherheitsbereich 54 für den Verlauf des Katheters 36. Durch eine Bestimmung der Punktionsposition im distalen Bereich 52 ist ein weiterer Punkt im Verlauf des Katheters bekannt, und der Unsicherheitsbereich 54 kann verkleinert werden.

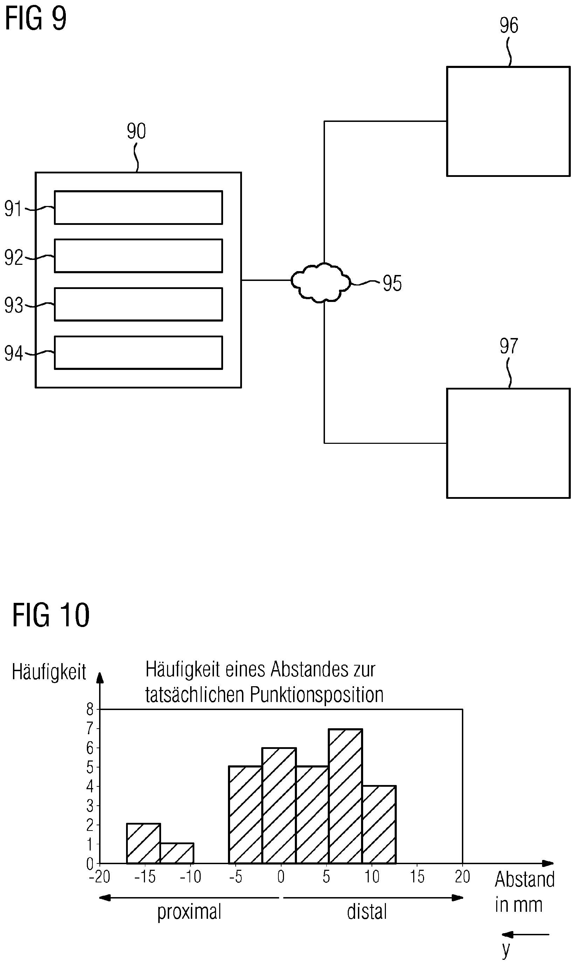

Weiterhin sind im dargestellten Ausführungsbeispiel eine erste Instrumentenposition 54 und eine zweite Instrumentenposition 55 dargestellt. Die erste Instrumentenposition 54 ist hierbei im zweiten Bilddatensatz 32 darstellbar, die zweite Instrumentenposition 55 ist nicht zweiten Bilddatensatz 32 enthalten und liegt im distalen Unsicherheitsbereich 54 des Verlaufs des Katheters 36. Die erste Instrumentenposition 54 und die zweite Instrumentenposition 55 sind hier jeweils dreidimensionale Instrumentenposition, im zweidimensionalen zweiten Bilddatensatz 32 sind jeweils nur Projektionen der ersten Instrumentenposition 54 und der zweiten Instrumentenposition 55 zu entnehmen. Das Bestimmen der ersten Instrumentenposition 54 und der zweiten Instrumentenposition werden in der Fig. 9 und der dazugehörigen Beschreibung näher erläutert.

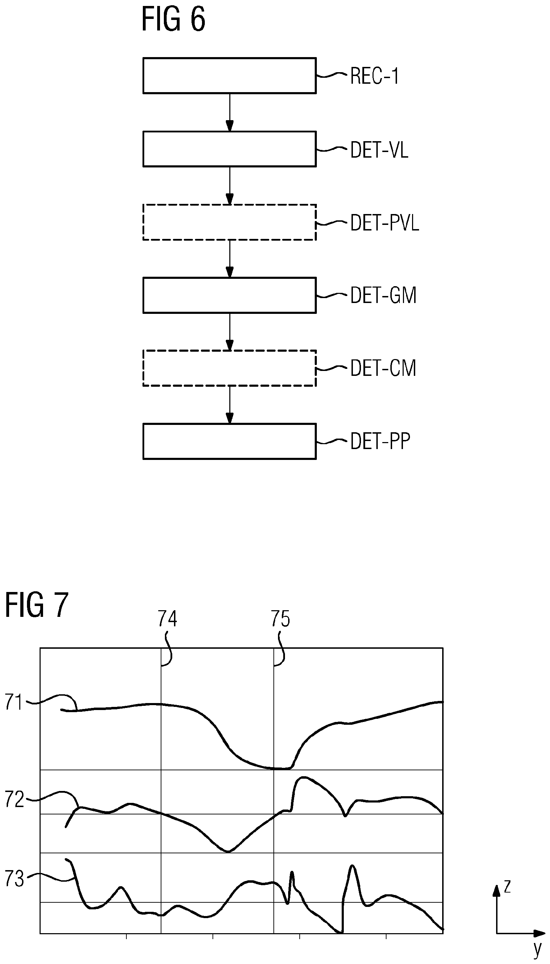

Fig. 6 zeigt ein Flussdiagramm eines ersten Ausführungsbeispiels des erfindungsgemäßen Verfahrens zum Bestimmen einer Punktionsposition.

Der erste Schritt des dargestellten Ausführungsbeispiels ist das Empfangen REC-1 eines ersten Bilddatenzes 31 eines Untersuchungsbereichs mittels einer Schnittstelle 91, wobei der erste Bilddatensatz 31 das Gefäß abbildet. Im dargestellten Ausführungsbeispiel ist der erste Bilddatensatz 31 ein dreidimensionaler Bilddatensatz, und der erste Bilddatensatz 31 umfasst die abdominale Aorta 11 eines Patienten 30 sowie die beiden gemeinsame Darmbeinarterien 12 des Patienten 30. Weiterhin umfasst der dreidimensionale erste Bilddatensatz 31 auch eine oder beide äußere Beckenarterien 13 sowie eine oder beide Oberschenkelarterien 14 des Patienten 30.

Der zweite Schritt des dargestellten Ausführungsbeispiels ist das Bestimmen DET-VL einer Gefäßlinie 37 des Gefäßes basierend auf dem ersten Bilddatensatz 31 mittels einer Recheneinheit 92. Methoden zur Bestimmung der Gefäßlinie 37 sind hierbei dem Fachmann bekannt, beispielsweise aus der Druckschrift V. R. Goel et al., "Automated Vascular Geometrie Analysis of Aortic Aneurysms", IEEE Comput. Graph Appl. 2008, Jahrgang 28, Heft Nr. 3, Seiten 76-86.

Der dritte Schritt des dargestellten Ausführungsbeispiels ist das Bestimmen DET-PVL einer projizierten Gefäßlinie 71 durch Projektion 34 der Gefäßlinie 37 entlang einer ersten Richtung x mittels der Recheneinheit 92. Diese projizierte Gefäßlinie 71 wird verwendet werden, um ein Steigungsmaß 72 basierend auf der Gefäßlinie 37 zu bestimmen. Im dargestellten Ausführungsbeispiel ist die Gefäßlinie 37 durch eine Menge von dreidimensionalen Voxeln im ersten Bilddatensatz 31 gegeben, wobei jeder der Voxel eine Koordinate bezüglich der ersten Richtung x, einer zweiten Richtung y und einer dritten Richtung z aufweist. Die bezüglich der ersten Richtung projizierte Gefäßlinie 71 ist dann durch eine Menge von Pixeln gegeben, die durch Weglassen der Koordinaten der Gefäßlinienpunkte bezüglich der ersten Richtung x entstehen und nur noch eine Koordinate bezüglich der zweiten Richtung y und eine Koordinate bezüglich der dritten Richtung aufweisen. Entspricht die erste Richtung x nicht einer der Koordinatenachsen, dann kann ein allgemeiner Projektionsoperator auf die Koordinaten der Punkte angewendet werden. Die projizierte Gefäßlinie 71 kann dann auch im Allgemeinen in eine implizite diskrete Form z(y) übergeführt werden. Es ist dann auch optional und vorteilhafterweise möglich, die Pixel der projizierten Gefäßlinie 71 zu interpolieren, um zu einer durchgehenden projizierten Gefäßlinie 71 oder zu einer differenzierbaren projizierten Gefäßlinie 71 zu gelangen.

Alternativ kann die Gefäßlinie 37 auch durch eine Kurve im dreidimensionalen Raum gegeben sein, also durch eine Funktion $\mathbb{R} \to \mathbb{R}^3$, $s \to (x(s), y(s), z(s))$ die einen Kurvenparameter s auf eine dreidimensionale Koordinate abbildet. Die projizierte Gefäßlinie 71 ist dann durch eine Kurve im zweidimensionalen Raum gegeben, die durch Anwendung eines Projektionsoperators auf die dreidimensionale Kurve hervorgeht, beispielsweise durch die Funktion $\mathbb{R} \to \mathbb{R}^2$, $s \to (y(s), z(s))$. Diese projizierte Gefäßlinie 71 kann dann auch im Allgemeinen in eine implizite Form z(y) übergeführt werden. Es ist natürlich auch möglich, durch die Wahl eines beliebigen Projektionsoperators in jede beliebige Richtung zu projizieren.

Das Bestimmen DET-PVL der projizierten Gefäßlinie 71 ist ein optionaler Schritt. Dieser Schritt kann insbesondere entfallen, wenn das Steigungsmaß 72 und das Krümmungsmaß 73 nicht basierend auf der projizierten Gefäßlinie 71 berechnet werden sollen, sondern jeweils basierend auf einem Tangentenvektor der Gefäßlinie 37.

Der vierte Schritt des dargestellten Ausführungsbeispiels ist das Bestimmen DET-GM eines Steigungsmaßes 72 der projizierten Gefäßlinie 71 bezüglich einer zweiten Richtung y mittels der Recheneinheit 92, wobei die zweite Richtung y orthogonal zur ersten Richtung x ist.

Im dargestellten Ausführungsbeispiel wird als Steigungsmaß 72 eine diskrete erste Ableitung (ein anderes Wort ist Differenzenquotient) der projizierten Gefäßlinie 71 verwendet

$$z'_d(y) = \frac{z(y + h) - z(y)}{h}$$

wobei der Pixelabstand h in der Regel als h = 1 gewählt wird. Insbesondere wenn die projizierte Gefäßlinie 71 eine glatte Funktion ist, kann auch die kontinuierliche erste Ableitung (ein anderes Wort ist Differentialquotient) z'(y) als Steigungsmaß 72 verwendet werden. Optional kann eine diskrete erste Ableitung durch Filterung geglättet werden, um den Einfluss von Rauschen zu verringern. Bei diesem Steigungsmaß 72 handelt es sich um ein Steigungsmaß 72 bezüglich der zweiten Richtung y. Das Steigungsmaß 72 basiert also insbesondere auf der Gefäßlinie 37.

Alternativ kann ein Steigungsmaß 72 kann auch basierend auf einem Tangentenvektor der Gefäßlinie 37 bestimmt werden. Ist die Gefäßlinie 37 durch eine Menge von Punkten $p_1, ..., p_N$ gegeben, dann entspricht der Tangentenvektor $t_i$ in einem Punkt $p_i$ einem Differenzvektor von zwei Punkten der Gefäßlinie 37, beispielsweise $t_i = (p_{i+1} - p_i)/|p_{i+1} - p_i|$ oder $t_i = (p_{i+1} - p_{i-1})/|p_{i+1} - p_{i-1}|$, wobei $|p_i - p_j|$ der euklidische Abstand zwischen den Punkten $p_i$ und $p_j$ ist. Dies kann als diskrete erste Ableitung bezüglich des Kurvenparameters aufgefasst werden, der hier als Index verwendet wird. Ist die Gefäßlinie 37 durch eine differenzierbare Kurve g(s) = (x(s), y(s), z(s)) gegeben, dann kann der Tangentenvektor $t(s_0)$ in einem Punkt $g(s_0)$ beispielsweise durch die komponentenweise Ableitung nach dem Kurvenparameter s gegeben sein:

$$t(s_0) = \left( \left.\frac{dx(s)}{ds}\right|_{s_0}, \left.\frac{dy(s)}{ds}\right|_{s_0}, \left.\frac{dz(s)}{ds}\right|_{s_0} \right)$$

Basierend auf dem Tangentenvektor kann ein skalares Steigungsmaß 72 in einem Punkt durch skalare Multiplikation des Tangentenvektors mit einem Einheitsvektor berechnet werden, wobei der Einheitsvektor in die vorgegebene Richtung zeigt. Beispielsweise kann ein Steigungsmaß 72 gegeben sein durch $t_i$ o $e_3$ bzw. $t(s)$ o $e_3$, wobei $e_2 = (0, 0, 3)$ ein Einheitsvektor in die dritte Richtung z ist.

Ein fünfter und optionaler Schritt des dargestellten Ausführungsbeispiels ist das Bestimmen DET-CM eines Krümmungsmaßes 73 der projizierten Gefäßlinie 71 bezüglich der zweiten Richtung y mittels der Recheneinheit 92. Das Krümmungsmaß 73 basiert also insbesondere auf der Gefäßlinie 37.

Im dargestellten Ausführungsbeispiel wird als Krümmungsmaßes 73 eine diskrete zweite Ableitung der projizierten Gefäßlinie 71 verwendet:

$$z''_d(y) = \frac{z(y + h) - 2z(y) + z(y - h)}{h^2}$$

wobei der Pixelabstand h in der Regel als h = 1 gewählt wird. Insbesondere wenn die projizierte Gefäßlinie 71 eine glatte Funktion ist, kann auch die kontinuierliche zweite Ableitung (ein anderes Wort ist Differentialquotient) z"(y) als Krümmungsmaß 73 verwendet werden. Optional kann eine diskrete zweite Ableitung durch Filterung geglättet werden, um den Einfluss von Rauschen zu verringern. Bei diesem Krümmungsmaß 73 handelt es sich um ein Krümmungsmaß 73 bezüglich der zweiten Richtung y.

Alternativ kann ein Krümmungsmaß 73 kann auch basierend auf einem Tangentenvektor der Gefäßlinie 37 bestimmt werden, insbesondere auf einer Differenz von zwei Tangentenvektoren und/oder insbesondere basierend auf einem Krümmungsvektor. Ist die Gefäßlinie 37 durch eine Menge von Punkten $p_1, ..., p_N$ gegeben, dann kann ein Krümmungsmaß 73 durch skalare Multiplikation eines Einheitsvektors mit einem Krümmungsvektor ki = $(t_{i+1} - t_i)/|p_{i+1} - p_i|$ oder $k_i = (t_{i+1} - t_{i-1})/|p_{i+1} - p_{i-1}|$ bestimmt werden. Dies kann als diskrete zweite Ableitung bezüglich des Kurvenparameters aufgefasst werden, der hier als Index verwendet wird. Ist die Gefäßlinie 37 durch eine differenzierbare Kurve $g(s) = (x(s), y(s), z(s))$ gegeben, dann kann der Krümmungsvektor $k(s_0)$ in einem Punkt $g(s_0)$ beispielsweise durch die komponentenweise zweite Ableitung nach dem Kurvenparameter s gegeben sein:

$$k(s_0) = \left( \frac{d^2 x(s)}{ds^2}\bigg|_{s_0}, \frac{d^2 y(s)}{ds^2}\bigg|_{s_0}, \frac{d^2 z(s)}{ds^2}\bigg|_{s_0} \right)$$

Insbesondere ist der Krümmungsvektor k die komponentenweise erste Ableitung des Tangentenvektors t nach dem Kurvenparameter s, also basiert auch der Krümmungsvektor k auf dem Tangentenvektor t der Gefäßlinie 37. Basierend auf dem Krümmungsvektor kann ein skalares Krümmungsmaß 73 in einem Punkt durch skalare Multiplikation des Krümmungsvektors mit einem Einheitsvektor berechnet werden. Beispielsweise kann ein Krümmungsmaß 73 gegeben sein durch $k_i$ o $e_3$ bzw. $k(s)$ o $e_3$, wobei $e_3 = (0, 0, 1)$ ein Einheitsvektor in die dritte Richtung z ist.

Der sechste Schritt des dargestellten Ausführungsbeispiels ist das Bestimmen DET-PP der Punktionsposition 74 des Gefäßes basierend auf dem Steigungsmaß 72 und dem Krümmungsmaß 73 mittels der Recheneinheit 92. Alternativ kann das Bestimmen DET-PP der Punktionsposition 74 auch nur auf dem Krümmungsmaß 73 basieren. Weiterhin alternativ kann das Bestimmen DET-PP der Punktionsposition 74 zusätzlich auf einem weiteren Extremum der Gefäßlinie 37 und/oder der projizierten Gefäßlinie 71 basieren. Das Bestimmen DET-PP der Punktionsposition 74 wird detailliert in der Fig. 7 sowie der zugehörigen Beschreibung dargestellt.

Fig. 7 zeigt eine projizierte Gefäßlinie 71, ein Steigungsmaß 72 und ein Krümmungsmaß 73. Beim Steigungsmaß 72 handelt es sich um eine diskrete erste Ableitung der projizierten Gefäßlinie 71 bezüglich der zweiten Richtung y, beim Krümmungsmaß 73 handelt es sich um eine diskrete zweite Ableitung der projizierten Gefäßlinie 71 bezüglich der zweiten Richtung. Das hier beschriebene Vorgehen ist aber auch analog anwendbar, wenn das Steigungsmaß 72 und/oder das Krümmungsmaß 73 auf einem Tangentenvektor der Gefäßlinie 37 basieren.

Weiterhin ist in der Fig. 7 ein Extremum 75 abgebildet, welches das globale Minimum der projizierten Gefäßlinie 71 ist. Dieses globale Minimum kann basierend alleine auf dem Steigungsmaß 72 bestimmt werden, indem für alle Nullstellen des Steigungsmaßes 72 der Wert der projizierten Gefäßlinie 71 bestimmt wird (wobei der Wert hier der Koordinate bezüglich der dritten Richtung z entspricht), und die Nullstelle mit dem kleinsten entsprechenden Wert der projizierten Gefäßlinie 71 als Extremum 75 bzw. globales Minimum verwendet wird. Weiterhin kann auch das Vorzeichen des Krümmungsmaßes 73 verwendet werden, um zwischen einem Minimum und einem Maximum der projizierten Gefäßlinie 71 zu unterscheiden.

Das Extremum 75 kann dann verwendet werden, um den Punktionspunkt 74 einfacher zu bestimmen, in diesem Fall ist der Punktionspunkt 74 das Maximum distal vom Extremum 75, welches bezüglich der zweiten Koordinate y den geringsten Abstand zum Extremum 75 aufweist. Alternativ kann die Punktionsposition 71 auch als globales Maximum distal des Extremums 75 bestimmt werden, wiederum alternativ kann die Punktionsposition 74 auch als globales Maximum der projizierten Gefäßlinie 71 bestimmt werden.

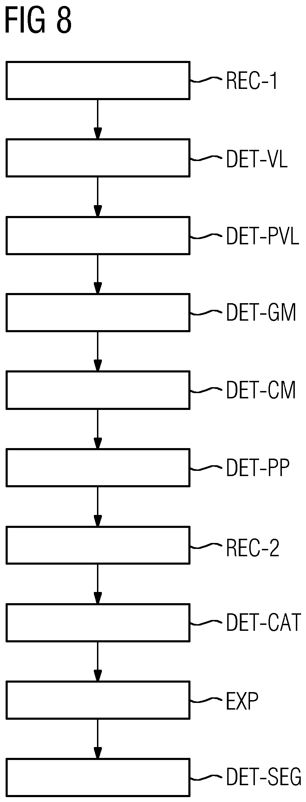

Fig. 8 zeigt ein zweites Ausführungsbeispiel des Verfahrens zum Bestimmen einer Punktionsposition 74. Die Schritte des Empfangens REC-1 eines ersten Bilddatensatzes 31, des Bestimmens DET-VL einer Gefäßlinie 37, des Bestimmens DET-PVL einer projizierten Gefäßlinie 71, des Bestimmens DET-GM eines Steigungsmaßes 72, des Bestimmens DET-GM eines Krümmungsmaßes 73 und des Bestimmens DET-PP der Punktionsposition 74 sind identisch zum Ausführungsbeispiel der Fig. 6. Insbesondere können die genannten Schritte die vorteilhaften Aus- und Weiterbildungen aufweisen, die in der Beschreibung zur Fig. 6 genannt wurden.

Der siebte Schritt des dargestellten Ausführungsbeispiels ist das Empfangen REC-2D eines zweiten Bilddatensatzes 32 des Untersuchungsbereichs mittels der Schnittstelle 91, wobei der zweite Bilddatensatz 32 einen Katheter 36 im Untersuchungsbereich abbildet. In diesem Ausführungsbeispiel ist der zweite Bilddatensatz 32 ein zweidimensionaler Bilddatensatz, der mittels einer zweiten bildgebenden medizinischen Vorrichtung 96 aufgenommen wurde. Beispielsweise kann es sich bei dem zweiten Bilddatensatz 32 um den in Fig. 5 dargestellten Bilddatensatz handeln. Der zweite Bilddatensatz 32 wird insbesondere während einer Katheranwendung aufgenommen, mit anderen Worten also zu einem Zeitpunkt, zu dem ein Katheter 36 in das Gefäß eingeführt ist.

Der achte Schritt des dargestellten Ausführungsbeispiels ist das Bestimmen DET-CAT einer ersten Instrumentenposition 54 basierend auf dem zweiten Bilddatensatz 32. Im dargestellten Ausführungsbeispiel ist die erste Instrumentenposition 54 eine dreidimensionale Position, welche insbesondere durch die Angabe von jeweils einer Koordinate bezüglich der ersten Richtung x, der zweiten Richtung y und der dritten Richtung z gegeben ist. Handelt es sich, wie im dargestellten Ausführungsbeispiel, beim zweiten Bilddatensatz 32 um einen zweidimensionalen Bilddatensatz, kann dieser mindestens zwei zweidimensionale Bilder umfassen, wobei diese beiden zweidimensionalen Bilder aus unterschiedlichen Richtungen aufgenommen wurden, um aus den mindestens zwei zweidimensionalen Koordinaten eine dreidimensionale Position zu rekonstruieren. Alternativ ist aus der Druckschrift US 20150094567 A1 auch bekannt, die erste Instrumentenposition basierend auf nur einem zweidimensionalen Bild zu bestimmen. Ist alternativ der zweite Bilddatensatz 32 ein dreidimensionaler Bilddatensatz, können die Koordinaten der ersten Instrumentenposition 54 direkt aus dem zweiten Bilddatensatz 32 abgelesen werden.

Alternativ kann die erste Instrumentenposition 54 auch eine zweidimensionale Position sein, welche insbesondere durch die Angabe von jeweils einer Koordinate bezüglich der zweiten Richtung y und der dritten Richtung z gegeben ist. Handelt es sich dann beim zweiten Bilddatensatz 32 um einen zweidimensionalen Bilddatensatz, kann die erste Instrumentenposition 54 direkt aus dem zweiten Bilddatensatz 32 abgelesen werden. Handelt es sich alternativ beim zweiten Bilddatensatz 32 um einen dreidimensionalen Bilddatensatz, kann die zweidimensionale erste Instrumentenposition 54 durch eine Projektion einer direkt ablesbaren dreidimensionalen Instrumentenposition bestimmt werden, insbesondere durch Projektion bezüglich der ersten Richtung x.

Der neunte Schritt des dargestellten Ausführungsbeispiels ist das Extrapolieren EXP einer zweiten Instrumentenposition 55 basierend auf der ersten Instrumentenposition 54 und der Punktionsposition 74. In diesem Ausführungsbeispiel wird hierzu eine Kurve durch die erste Instrumentenposition 54 und die Punktionsposition 74 bestimmt (durch Bestimmung der Parameter des Polynoms), welche den Verlauf des Gefäßes approximiert. Die zweite Instrumentenposition 55 ist dann ein Punkt dieser Kurve zwischen der ersten Instrumentenposition 54 und der Punktionsposition 74. Hierbei kann als Kurve eine Gerade durch die erste Instrumentenposition und die Punktionsposition 74 gewählt werden, alternativ kann unter Verwendung von Annahmen über eine Krümmung des Gefäßes auch andere Kurven, wie z.B. Polynome höheren Grades, Splines oder NURBS (Akronym für "nicht-uniforme rationale B-Splines", der englische Fachbegriff ist "Non-uniform rational B-Splines") durch die erste Instrumentenposition 54 und die Punktionsposition 74 gewählt werden.

Vorteilhafterweise können auch mehrere erste Instrumentenposition 54 verwendet werden, um eine zweite Instrumentenposition 55 zu bestimmen. Ist das Gleichungssystem zur Bestimmung der Parameter der Kurve überbestimmt, können diese durch eine Ausgleichsrechnung (der englische Fachbegriff ist "fitting"), beispielsweise basierend auf der Methode der kleinsten Quadrate, bestimmt werden. Vorteilhafterweise kann die zweite Instrumentenposition 55 auch auf einer oder mehreren ersten Instrumentenrichtungen basieren, beispielsweise auf den Tangentenvektoren des Katheters 36 in der einen oder den mehreren ersten Instrumentenposition 54.

Der zehnte Schritt des dargestellten Ausführungsbeispiels ist das Bestimmen DET-SEG einer zweiten Segmentierung des Gefäßes basierend auf der ersten Segmentierung 35, der Punktionsposition 74 des Gefäßes und dem zweiten Bilddatensatz 32 mittels der Recheneinheit 92. Hierbei erfolgt das das Bestimmen (ET-SEG)der zweiten Segmentierung derart, dass die erste Instrumentenposition 54 und die zweite Instrumentenposition 55 innerhalb der zweiten Segmentierung des Gefäßes angeordnet sind.

Fig. 9 zeigt eine Positionsbestimmungseinheit 90 zur Bestimmung einer Punktionsposition. Die hier gezeigte Positionsbestimmungseinheit 90 ist ausgelegt, ein erfindungsgemäßes Verfahren auszuführen. Diese Positionsbestimmungseinheit 90 umfasst eine Schnittstelle 91, eine Recheneinheit 92, eine Speichereinheit 93 sowie eine Ein- und Ausgabeeinheit 94.

Bei der Positionsbestimmungseinheit 90 kann es sich insbesondere um einen Computer, eine Workstation, einen Mikrocontroller oder um einen integrierten Schaltkreis handeln. Alternativ kann es sich bei der Positionsbestimmungseinheit 90 um einen realen oder virtuellen Verbund von Computern handeln (ein englischer Fachbegriff für einen realen Verbund ist "Cluster", ein englischer Fachbegriff für einen virtuellen Verbund ist "Cloud").

Bei einer Schnittstelle 91 kann es sich um eine Hardware- oder Softwareschnittstelle handeln (beispielsweise PCI-Bus, USB oder Firewire). Eine Recheneinheit 92 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit 93 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Eine Ein- und Ausgabeeinheit 94 umfasst wenigstens eine Eingabeeinheit und/oder wenigstens eine Ausgabeeinheit.

Die Positionsbestimmungseinheit 90 ist in diesem Ausführungsbeispiel über ein Netzwerk 94 mit einer ersten bildgebenden medizinischen Vorrichtung 95 und einer zweite bildgebenden medizinischen Vorrichtung 96 verbunden. Beim Netzwerk 94 kann es sich um ein lokales Netzwerk (ein englischer Fachbegriff ist "Local Area Network", kurz "LAN") oder um ein großräumiges Netzwerk (ein englischer Fachbegriff ist "Wide Area Network", kurz "WAN") handeln. Ein Beispiel für ein lokales Netzwerk ist ein Intranet, ein Beispiel für ein großräumiges Netzwerk ist das Internet. Das Netzwerk 94 kann insbesondere auch drahtlos ausgeführt sein, insbesondere als WLAN (für "wireless LAN", im Englischen ist die Abkürzung "WiFi" gebräuchlich) oder als Bluetooth-Verbindung. Das Netzwerk 94 kann auch als Kombination der genannten Beispiele ausgeführt sein.

Alternativ ist es auch möglich, dass die Positionsbestimmungseinheit 90 über ein erstes Netzwerk mit der ersten bildgebenden medizinischen Vorrichtung 95 verbunden ist, und über ein zweites Netzwerk mit der zweiten bildgebenden medizinischen Vorrichtung 96 verbunden ist, wobei das erste und das zweite Netzwerk die Aus- und Weiterbildungen des Netzwerks 94 aufweisen können. Alternativ ist es auch möglich, dass die Positionsbestimmungseinheit 90 als Teil der ersten bildgebenden Vorrichtung 95 ausgebildet ist, und die erste bildgebende Vorrichtung 95 mit der zweiten bildgebenden Vorrichtung 96 (optional über ein Netzwerk) verbunden ist. Alternativ ist es auch möglich, dass die Positionsbestimmungseinheit 90 als Teil der zweiten bildgebenden Vorrichtung 96 ausgebildet ist, und die zweite bildgebende Vorrichtung 96 mit der ersten bildgebenden Vorrichtung 95 (optional über ein Netzwerk) verbunden ist. Eine Verbindung (optional über ein Netzwerk) ist im Sinne dieser Ausführungen auch gegeben, wenn die erste oder die zweite bildgebende medizinische Vorrichtung 95, 96 den ersten Bilddatensatz 31 bzw. den zweiten Bilddatensatz 32 in einer zentralen Speichereinheit speichert, und die Positionsbestimmungseinheit 90 auf diese zentralen Speichereinheit zugreift. Bei dieser zentralen Speichereinheit kann es sich insbesondere um ein PACS (engl. Akronym für "Picture Archiving and Communication System"), um ein RIS (engl. Akronym für "Radiology Information System") oder um ein HIS (engl. Akronym für "Hosptial Information System") handeln. In allen dargestellten Alternativen kann eine Verbindung auch nur mit einer der beiden bildgebenden Modalitäten 95, 96 vorliegen.

Die erste bildgebende medizinische Vorrichtung 95 ist dazu ausgebildet, einen ersten Bilddatensatz 31 aufzunehmen. Ist der erste Bilddatensatz 31 ein dreidimensionaler Bilddatensatz, kann es sich bei der ersten bildgebenden medizinischen Vorrichtung insbesondere um einen Computertomographen, um einen Magnetresonanztomographen oder um ein C-Bogen-Röntgengerät handeln, wobei das C-Bogen-Röntgengerät dazu ausgebildet ist, aus mehreren Richtung zweidimensionale Röntgenbilder aufzunehmen und diese zu einem dreidimensionalen Bilddatensatz zu rekonstruieren. Ist der erste Bilddatensatz 31 ein zweidimensionaler Bilddatensatz, kann es sich bei der ersten bildgebenden medizinischen Vorrichtung 95 um ein Röntgendurchleuchtungsgerät, insbesondere um ein C-Bogen-Röntgengerät handeln. Bei der Aufnahme des ersten Bilddatensatzes 31 mit der ersten bildgebenden medizinischen Vorrichtung 95 kann insbesondere ein Röntgenkontrastmittel verwendet werden, wenn die Aufnahme mittels Röntgenstrahlung erfolgt.

Die zweite bildgebende medizinische Vorrichtung 95 ist dazu ausgebildet, einen zweiten Bilddatensatz 32 aufzunehmen. Bei der zweiten bildgebenden medizinischen Vorrichtung 96 kann es sich insbesondere um Röntgendurchleuchtungsgerät, insbesondere um ein C-Bogen-Röntgengerät handeln. Bei der Aufnahme des zweiten Bilddatensatzes 32 mit der zweiten bildgebenden medizinischen Vorrichtung 96 kann insbesondere ein Röntgenkontrastmittel verwendet werden, wenn die Aufnahme mittels Röntgenstrahlung erfolgt.

Fig. 11 zeigt experimentelle Daten, die sich aus der Anwendung des dargestellten Verfahrens zur Bestimmung einer Punktionsposition ergeben. Hierbei wurde basierend auf fünfzehn dreidimensionalen Bilddatensätzen von fünfzehn Patienten mit dem erfindungsgemäßen Verfahren beidseitig (also insgesamt dreißigmal) die Punktionsposition bestimmt, wobei es sich bei jedem der dreidimensionalen Bilddatensätzen um eine Computertomographieaufnahme handelt. Als Vergleich wurde für jeden der dreidimensionalen Bilddatensätze eine Vergleichsposition durch eine manuelle Annotation des dreidimensionalen Bilddatensatzes durch einen Radiologen ermittelt. Im Histogramm ist die relative Häufigkeit der entstehenden Abweichungen zwischen der durch das Verfahren ermittelten Punktionsposition und der Vergleichsposition aufgetragen. Der mittlere Betrag der Abweichung ergibt sich zu 6.3 mm $\pm$ 4.5 mm, in 24 der 30 Datensätze ergibt sich eine Abweichung von unter 10 mm. Die maximale Abweichung ergibt sich zu 18 mm. Zur Einschätzung der Genauigkeit kann der Durchmesser eines Katheters 36 zur Behandlung eines abdominellen Aorten-Aneurysmas herangezogen werden, der 7 mm beträgt.

**Patentansprüche**

1. Verfahren zum Bestimmen einer Punktionsposition (74) eines Gefäßes, umfassend die folgenden Verfahrensschritte:

   - Empfangen (REC-1) eines ersten Bilddatensatzes (31) eines Untersuchungsbereichs mittels einer Schnittstelle (91), wobei der erste Bilddatensatz (31) das Gefäß abbildet,
   - Bestimmen (DET-VL) einer Gefäßlinie (37) des Gefäßes basierend auf dem ersten Bilddatensatz (31) mittels einer Recheneinheit (92),
   - Bestimmen (DET-GM) eines Steigungsmaßes (72) basierend auf der Gefäßlinie (37) mittels der Recheneinheit (92),
   - Bestimmen (DET-PP) der Punktionsposition (74) des Gefäßes basierend auf dem Steigungsmaß (72) mittels der Recheneinheit (92),

   wobei die Punktionsposition (74) ein Punkt im ersten Bilddatensatz ist, der eine voraussichtliche Stelle des Eindringens eines Katheters in das Gefäß darstellt.

2. Verfahren nach dem Anspruch 1, weiterhin umfassend den folgenden Verfahrensschritt:

   - Bestimmen (DET-PVL) einer projizierten Gefäßlinie (71) durch Projektion der Gefäßlinie (37) entlang einer ersten Richtung (x) mittels der Recheneinheit (92),

   wobei das Steigungsmaß (72) ein Steigungsmaß (72) der projizierten Gefäßlinie (71) bezüglich einer zweiten Richtung (y) ist, und wobei die zweite Richtung (y) orthogonal zur ersten Richtung (x) ist.

3. Verfahren nach dem Anspruch 1, wobei das Steigungsmaß (72) auf einem Tangentenvektor der Gefäßlinie (37) basiert.

4. Verfahren nach einem der vorstehenden Ansprüche, wobei die Punktionsposition (74) basierend auf einer Nullstelle des Steigungsmaßes (72) bestimmt wird.

5. Verfahren nach einem der vorstehenden Ansprüche, weiterhin umfassend den folgenden Verfahrensschritt:

   - Bestimmen (DET-CM) eines Krümmungsmaßes (73) basierend auf der Gefäßlinie (37) mittels der Recheneinheit (92);

   wobei das Bestimmen (DET-PP) der Punktionsposition (74) des Gefäßes weiterhin auf dem Krümmungsmaß (73) basiert.

6. Verfahren nach einem der vorstehenden Ansprüche, wobei das Steigungsmaß (72) eine erste Ableitung ist.

7. Verfahren nach einem der vorstehenden Ansprüche, wobei das Bestimmen (DET-PP) der Punktionsposition (74) weiterhin auf einem Extremum (75) der Gefäßlinie (37) basiert,
   wobei sich die Koordinaten des Extremums (75) von den Koordinaten der Punktionsposition (74) unterscheiden.

8. Verfahren nach dem Anspruch 7, wobei die Punktionsposition (74) bezüglich des Extremums (75) distal oder proximal gelegen ist.

9. Positionsbestimmungseinheit (90) zum Bestimmen einer Punktionsposition (74), umfassend folgende Einheiten:

   - Schnittstelle (91), ausgebildet zum Empfangen (REC-3D) eines ersten Bilddatensatzes (31) eines Untersuchungsbereichs, wobei der erste Bilddatensatz (31) das Gefäß abbildet,
   - Recheneinheit (92), ausgebildet zum Bestimmen (DET-VL) einer Gefäßlinie (37) des Gefäßes basierend auf dem ersten Bilddatensatz (31),
   weiterhin ausgebildet zum Bestimmen (DET-GM) eines Steigungsmaßes (72) basierend auf der Gefäßlinie (37),

weiterhin ausgebildet zum Bestimmen (DET-PP) der Punktionsposition (74) des Gefäßes basierend auf dem Steigungsmaß (72),

wobei die Punktionsposition (74) ein Punkt im ersten Bilddatensatz ist, der eine voraussichtliche Stelle des Eindringens eines Katheters in das Gefäß darstellt.

**10.** Positionsbestimmungseinheit (90) nach Anspruch 9, weiterhin dazu ausgebildet ein Verfahren nach einem der Ansprüche 2 bis 8 auszuführen.

**11.** Bildgebende medizinische Vorrichtung (94, 95), umfassend eine Positionsbestimmungseinheit (90) nach Anspruch 9 oder 10.

**12.** Computerprogrammprodukt mit einem Computerprogramm, welches direkt in eine Speichereinheit (93) einer Positionsbestimmungseinheit (90) ladbar ist, mit Programmabschnitten, um alle Schritte des Verfahrens nach einem der Ansprüche 1 bis 8 auszuführen, wenn die Programmabschnitte von der Positionsbestimmungseinheit (90) ausgeführt werden.

**13.** Computerlesbares Speichermedium, auf welchem von einer Positionsbestimmungseinheit (90) lesbare und ausführbare Programmabschnitte gespeichert sind, um alle Schritte des Verfahrens nach einem der Ansprüche 1 bis 8 auszuführen, wenn die Programmabschnitte von der Positionsbestimmungseinheit (90) ausgeführt werden.

**Claims**

**1.** Method for determining a vessel puncture position (74) comprising the following method steps:

- reception (REC-1) of a first image dataset (31) of a region of interest by means of an interface (91),

wherein the first image dataset (31) maps the vessel,

- determination (DET-VL) of a vessel line (37) of the vessel based on the first image dataset (31) by means of a computing unit (92),
- determination (DET-GM) of a gradient measure (72) based on the vessel line (37) by means of the computing unit (92),
- determination (DET-PP) of the puncture position (74) of the vessel based on the gradient measure (72) by means of the computing unit (92),

wherein the puncture position (74) is a point in the first image dataset describing a probable location at which a catheter penetrates the vessel.

**2.** Method according to claim 1, further comprising the following method step:

- determination (DET-PVL) of a projected vessel line (71) by projection of the vessel line (37) along a first direction (x) by means of the computing unit (92),

wherein the gradient measure (72) is a gradient measure (72) of the projected vessel line (71) with respect to a second direction (y) and wherein the second direction (y) is orthogonal to the first direction (x).

**3.** Method according to claim 1, wherein the gradient measure (72) is based on a tangent vector of the vessel line (37).

**4.** Method according to one of the preceding claims, wherein the puncture position (74) is determined based on a zero point of the gradient measure (72).

**5.** Method according to one of the preceding claims, further comprising the following method step:

- determination (DET-CM) of a curvature measure (73) based on the vessel line (37) by means of the computing unit (92);

wherein the determination (DET-PP) of the puncture position (74) of the vessel is also based on the curvature measure (73) .

6. Method according to one of the preceding claims, wherein the gradient measure (72) is a first derivative.

7. Method according to one of the preceding claims, wherein the determination (DET-PP) of the puncture position (74) is further based on an extremum (75) of the vessel line (37), wherein the coordinates of the extremum (75) differ from the coordinates of the puncture position (74).

8. Method according to claim 7, wherein the puncture position (74) is situated distally or proximally to the extremum (75).

9. Position-determining unit (90) for determining a puncture position (74), comprising the following units:

- interface (91) embodied to receive (REC-3D) a first image dataset (31) of a region of interest, wherein the first image dataset (31) maps the vessel,
- computing unit (92) embodied to determine (DET-VL) a vessel line (37) of the vessel based on the first image dataset (31), furthermore embodied to determine (DET-GM) a gradient measure (72) based on the vessel line (37),
furthermore embodied to determine (DET-PP) the vessel puncture position (74) based on the gradient measure (72),
wherein the puncture position (74) is a point in the first image dataset describing a probable location at which a catheter penetrates the vessel.

10. Position-determining unit (90) according to claim 9, furthermore embodied to carry out a method according to one of claims 2 to 8.

11. Imaging medical device (94, 95), comprising a position-determining unit (90) according to claim 9 or 10.

12. Computer-program product with a computer program, which can be loaded directly into a memory unit (93) of a position-determining unit (90), with program sections for executing all steps of the method according to one of claims 1 to 8 when the program sections are executed by the position-determining unit (90) .

13. A computer-readable storage medium on which program sections which can be read and executed by a position-determining unit (90) are stored in order to carry out all the steps of the method according to one of claims 1 to 8 when the program sections are executed by the position-determining unit (90) .

**Revendications**

1. Procédé de détermination d'une position (74) de ponction d'un vaisseau, comprenant les stades de procédé suivants :

- réception (REC-1) d'un premier ensemble (31) de données d'image d'une partie à examiner au moyen d'une interface (91), dans lequel le premier ensemble (31) de données d'image représente le faisceau,
- détermination (DET-VL) d'une ligne (37) du vaisseau reposant la premier ensemble (31) de données d'image au moyen d'une unité (92) de calcul,
- détermination (DET-GM) d'une grandeur (72) de pente reposant sur la ligne (37) de vaisseau au moyen de l'unité (92) de calcul,
- détermination (DET-PP) de la position (74) de ponction du vaisseau reposant sur la grandeur (72) de pente au moyen de l'unité (92) de calcul,

dans lequel la position (74) de ponction est un point dans le premier ensemble de données d'image, qui représente un endroit prévisible de la pénétration d'un cathéter dans le vaisseau.

2. Procédé suivant la revendication 1, comprenant en outre le stade de procédé suivant :

- détermination (DET-PVL) d'une ligne (71) de vaisseau projeté par projection de la ligne (37) de vaisseau, suivant une première direction (x) au moyen de l'unité (92) de calcul,

dans lequel la grandeur (72) de pente est une grandeur (72) de pente de la ligne (71) de vaisseau projetée, rapportée à une deuxième direction (y), et dans lequel la deuxième direction (y) est orthogonale à la première direction (x).

3. Procédé suivant la revendication 1, dans lequel la grandeur (72) de pente repose sur un vecteur tangent de la ligne (37) de vaisseau.

4. Procédé suivant l'une des revendications précédentes, dans lequel on détermine la position (74) de ponction sur la base d'un point zéro de grandeur (72) de pente.

5. Procédé suivant l'une des revendications précédentes, comprenant en outre le stade de procédé suivant :

   - détermination (DET-CM) d'une grandeur (73) de courbure reposant sur la ligne (37) de vaisseau au moyen de l'unité (92) de calcul ;

   dans lequel la détermination (DET-PP) de la position (74) de ponction du faisceau repose en outre sur la grandeur (73) de courbure.

6. Procédé suivant l'une des revendications précédentes, dans lequel la grandeur (72) de pente est une dérivée première.

7. Procédé suivant l'une des revendications précédentes, dans lequel la détermination (DET-PP) de la position (74) de ponction repose en outre sur un extrémum (75) de la ligne (37) de vaisseau, dans lequel les coordonnées de l'extrémum (75) se distinguent des coordonnées de la position (74) de ponction.

8. Procédé suivant la revendication 7, dans lequel la position (74) de ponction est distale ou proximale de l'extrémum (75).

9. Unité (90) de détermination de position pour la détermination d'une position (74) de ponction, comprenant les unités suivantes :

   - une interface (91) constituée pour la réception (REC-3D) d'un premier ensemble (31) de données d'image d'une partie à examiner, dans lequel le premier ensemble (31) de données d'image représente le vaisseau,
   - l'unité (92) de calcul constituée pour la détermination (DET-VL) d'une ligne (37) du vaisseau reposant sur le premier ensemble (31) de données d'image,
   constituée en outre pour la détermination (DET-GM) d'une grandeur (72) de pente reposant sur la ligne (37) de vaisseau,
   constituée en outre pour la détermination (DET-PP) de la position (74) de ponction du vaisseau reposant sur la grandeur (72) de pente,
   dans lequel la position (74) de ponction est un point dans le premier ensemble de données d'image, qui représente un endroit prévisible de la pénétration d'un cathéter dans le vaisseau.

10. Unité (90) de détermination de position suivant la revendication 9, constituée en outre pour exécuter un procédé suivant l'une des revendications 2 à 8.

11. Installation (94, 95) médicale d'imagerie comprenant une unité (90) de détermination de position suivant la revendication 9 ou 10.

12. Produit de programme d'ordinateur comprenant un programme d'ordinateur, qui peut être chargé directement dans une unité (93) de mémoire d'une unité (90) de détermination de position comprenant des parties de programme pour effectuer tous les stades du procédé suivant l'une des revendications 1 à 8, lorsque les parties du programme sont réalisées par l'unité (90) de détermination de position.

13. Support de mémoire, déchiffrable par ordinateur, sur lequel sont mises en mémoire des parties de programme, déchiffrables et réalisables par une unité (90) de détermination de position, afin d'effectuer tous les stades du procédé suivant l'une des revendications 1 à 8, lorsque les parties de programme sont réalisées par l'unité (90) de détermination de position.

FIG 1

FIG 2

FIG 3

FIG 4

FIG 5

## FIG 6

## FIG 7

# FIG 8

| | Label |
|---|---|
| | REC-1 |
| | DET-VL |
| | DET-PVL |
| | DET-GM |
| | DET-CM |
| | DET-PP |
| | REC-2 |
| | DET-CAT |
| | EXP |
| | DET-SEG |

FIG 9

90

91
92
93
94

95

96

97

FIG 10

Häufigkeit

Häufigkeit eines Abstandes zur
tatsächlichen Punktionsposition

8
7
6
5
4
3
2
1
0

-20   -15   -10   -5   0   5   10   15   20

Abstand
in mm

proximal                          distal

y

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8929631 B2
- US 20150094567 A1

- EP 17164593

**In der Beschreibung aufgeführte Nicht-Patentliteratur**

- **GOEL V R et al.** Automated Vascular Geometrie Analysis of Aortic Aneurysms. *IEEE COMPUTER GRAPHICS AND APPLICATIONS,* Mai 2008, vol. 28 (3), 76-86

- **V. R. GOEL et al.** Automated Vascular Geometrie Analysis of Aortic Aneurysms. *IEEE Comput. Graph Appl.,* 2008, vol. 28 (3), 76-86